# EUROPÄISCHE PATENTANMELDUNG

(11) **EP 1 072 686 A2**
(43) Veröffentlichungstag der Anmeldung: **31.01.2001**
(21) Anmeldenummer: 00109388.9
(22) Anmeldetag: 03.05.2000
(51) Int. Cl.: C12Q 1/68

(54) **Verfahren zur 5'-Cap-abhängigen Anreicherung von cDNAs**

(30) Priorität: 05.05.1999 DE 19920611
(71) Anmelder: Roche Diagnostics GmbH, 68298 Mannheim (DE)
(72) Erfinder: Mueller, Manfred W., Prof. Dr., 3400 Klosterneuburg (AT); Schmidt, Wolfgang M., Dr., 1030 Wien (AT)

(57) **Zusammenfassung**

Der Erfindung betrifft ein Verfahren zur Modifikation, Klonierung und Amplifikation von an ihrem 5 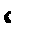 Ende vollständigen cDNAs, welches im wesentlichen dadurch gekennzeichnet ist, daß die Erststrang-cDNA-Synthese in Gegenwart von Mangan²⁺-Ionen durchgeführt wird oder Mangan²⁺ zeitlich versetzt als Additiv zugesetzt wird. Ausgelöst durch die CAP-Struktur am 5 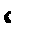 -Ende der revers transkribierten mRNA werden deoxy-Cytosine mit hoher Effizienz an das 3 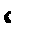 Ende der cDNA angefügt. In einer bevorzugten Ausführungsform wird im Anschluß an die Erststrang-cDNA Synthese mithilfe von Terminaler Transferase ein kontrolliertes Ribonukleotid-Tailing durchgeführt.

## Beschreibung

Die Erfindung entstammt dem Gebiet der Klonierung und Amplifikation von Nukleinsäuren und betrifft ein Verfahren zur Klonierung von am 5'-Ende vollständigen cDNAs.

Die molekulare Analyse von in vivo transkribierten messenger RNAs (mRNAs) erfolgt in der Regel durch Herstellung und Klonierung von sogenannten cDNAs. Dazu wird eine zuvor isolierte, poly-adenylierte mRNA unter Verwendung eines Oligo-dT-Primers zunächst revers transkribiert, d.h. in eine einzelsträngige, zur mRNA komplementäre cDNA umgeschrieben. Anschließend erfolgt mit dem Fachmann bekannten Methoden die Polymerisation eines zur einzelsträngigen cDNA komplementären Zweitstranges, sodaß eine doppelsträngige cDNA entsteht. Nach Amplifikation und Klonierung der cDNA oder Teilen der cDNA mit gängigen molekularbiologischen Methoden (vgl. Sambook, Molecular Cloning, Laboratory Manual, 2. Auflage, Kapitel 14) kann anschließend die Sequenz der mRNA bestimmt werden. Ein Nachteil dieser Methode liegt jedoch darin, daß die Identifikation und Klonierung von cDNAs, welche ein vollständiges 5'-Ende enthalten, selten oder nur mit einer sehr geringen Effizienz gelingt. Der Grund hierfür liegt in einer ineffizienten Reversen Transkriptase Reaktion aufgrund von sich ausbildenden intramolekularen Sekundärstrukturen sowie in einem häufig mengenmäßig begrenzten Ausgangsmaterial, dessen RNA Gehalt von nur unzureichender Qualität ist. Ein weiterer Nachteil besteht darin, daß bei konventionellen Methoden aufgrund der Art und Weise der Zweitstrangsynthese ein Verlust von terminalen 5 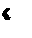 -Sequenzen der mRNA auftritt.

Verschiedene Ansätze, die in der Vergangenheit zur Überwindung dieses Problems entwickelt wurden, haben sich darauf konzentriert, die am 5 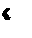 Ende von vollständigen cDNAs auftretende Cap-Struktur der mRNA als zusätzliches Selektionskritierium einzusetzen. Dabei handelt es sich um einen terminalen Guanosinrest, der an Position 7 methyliert und über eine 5 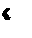 - 5 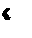 Binding mit der eigentlichen mRNA verknüpft ist.

Bei der "oligo capping" Technik (Maruyama and Sugaru, Gene 138, 171-174, 1994) wird die Cap Struktur zunächst durch eine geeignete Phosphatase-Behandlung enzymatisch entfernt und durch ein Oligonukleotid ersetzt, welches durch einen geeigneten Ligationsschritt mit dem 5 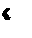 Ende der mRNA verbunden wird. Diese Methode erfordert jedoch eine Vielzahl enzymatischer Schritte sowie eine große Menge von Poly-A-mRNA als Ausgangsmaterial.

Ein anderes Verfahren, die Cap Finder-Methode (Clontech, Clontechniques 11, 1 (1996), Maleszka and Stange, Gene 202, 39-43(1997)), basiert auf einer marginalen Terminalen Transferase Aktivität der reversen Transkriptase, die zu einem sogenannten Template switching Effekt führt: Bei einer Erststrang cDNA Synthese werden unter geeigneten Bedingungen am 3 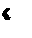 Ende der cDNA selektiv wenige deoxy-Cytosin-Reste durch die Reverse Transkriptase hinzugefügt. Dadurch wird eine Ankersequenz für ein sogenanntes Template switching Oligonukleotid mit einem aus Guanosin-Resten bestehenden 3 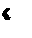 -Ende erzeugt, welches nach Hybridisierung an die Ankersequenz als Primer für die Zweitstrangsynthese der cDNA dient. Allerdings ist die offensichtlich an der 7-Methyl-Guanosin-Cap-Struktur der mRNA beginnende Terminale Transferase-Aktivität der Reversen Transkriptase, welche die Cytosin-Reste hinzufügt, bisher nicht näher charakterisiert, sodaß auch der Einfluß von bestimmten Sekundärstrukturen des mRNA-Templates oder des cDNA Endes auf die Template Switching Aktivität zum Zeitpunkt der Erfindung nicht weiter bekannt war und die Reaktion nach dem Stand der Technik nur mit geringer Effizienz durchgeführt werden konnte.

In einem alternativen Verfahren (WO 97/26368) erfolgt die Synthese einer geeigneten Ankersequenz mithilfe eines von der Reversen Transkriptase verschiedenen Terminalen Transferase Enzyms, wobei (anstelle von deoxy-Ribonukleotiden) zwei bis vier Ribonukleotide als Ankersequenz an das 3 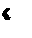 Ende der Erststrang-cDNA angefügt werden. Diese Methode besitzt allerdings den Nachteil, daß die cDNASynthese nicht selektiv für 5 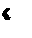 -Cap aufweisende mRNAs durchgeführt werden kann.

Die der Erfindung zugrunde liegende technische Aufgabe bestand demnach darin, ein weiteres Verfahren zur Modifikation, Klonierung oder Amplifikation von cDNAs zu entwickeln, mit dem auf möglichst einfache und effiziente Art und Weise cDNAs erhalten werden, die ein vollständiges 5 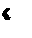 Ende aufweisen.

Diese technische Aufgabe wird dadurch gelöst, daß die Terminale Transferase-Reaktion zur Elongation der Erststrang-cDNA mit deoxy-Cytosinen durch eine Reverse Transkriptase sowohl in Gegenwart von Magnesium²⁺-Ionen als auch in Gegenwart von Mangan²⁺-Ionen durchgeführt wird. Die einzusetzende Mangankonzentration beträgt dabei vorzugsweise 1-20 mM, und unter optimierten Bedingungen 8 mM.

In einer Ausführungsform können die Mangan²⁺-Ionen bereits während der cDNA-Erststrangsynthese im verwendeten Puffersystem enthalten sein. Alternativ kann direkt nach einer in Abwesenheit von Mangan²⁺-Ionen erfolgten Erststrang-cDNA-Synthese eine Inkubation in Gegenwart von Mangan²⁺-Ionen erfolgen, wobei die ursprünglich eingesetzte Reverse Transkriptase noch unter diesen Bedingungen aktiv ist

Dabei entfaltet die reverse Transkriptase eine Terminale Transferase-Aktivität, die zu einer effizienten Addition von 2 bis 4 deoxy-Cytosinresten an den 3'-Terminus der neu synthetisierten cDNA führt. Diese Reaktion ist äußerst effizient und zu dem abhängig von der Anwesenheit der Cap-Struktur am 5'-Ende der mRNA Matrize.

In beiden Ausführungsformen bewirkt das erfindungsgemäße deoxy-Nukleotid-Tailing in Gegenwart von Mn²⁺-Ionen nicht nur eine hohe Effizienz der Tailing-Reaktion. Gleichzeitig bleibt die Spezifität der Reaktion - die Addition von zwei bis vier deoxy-Cytosinresten in Gegenwart einer 5 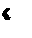 -Cap-Struktur an der m-RNA-Matritze - erhalten.

Beliebige Reverse Transkriptase-Enzyme können verwendet werden, mit der einzigen Einschränkung, daß das verwendete Enzym keine RNAseH Aktivität aufweisen darf.

Als Primer für die Erststrangsynthese wird vorzugsweise ein sogenannter "Ankerprimer" eingesetzt, welcher aus zwei Teilen besteht: Am 5'-Ende befindet sich eine sogenannte "Ankersequenz", die als Targetsequenz für PCR-Primer in zu einem späteren Zeitpunkt stattfindenden Amplifikationsreaktionen dienen kann. Das 3'-terminale Ende besteht dagegen aus einer Sequenz, die mit der zu identifizierenden mRNA hybridisieren kann. Dabei kann es sich beispielsweise um eine Oligo-dT-Sequenz handeln, welche mit dem poly-A-Schwanz 3'-Ende der mRNA hybridisiert.

In einer bevorzugten Ausführungsform wird im Anschluß an eine erfindungsgemäße cDNA Synthese in Anwesenheit einer von einer Reversen Transkriptase verschiedenen Terminalen Transferase die Erststrang-cDNA mit einem Ribonukleotidtriphosphat oder vergleichbaren Derivaten wie beispielsweise 2 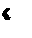 -O-Methyl- oder 2 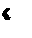 -O-Amino-Nukleotidtriphosphaten umgesetzt, sodaß an ihrem 3 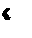 Ende eine Ankersequenz aus Ribonukleotid-Resten entsteht. Bei Einsatz eines bestimmten Ribonukleotid-Triphosphats wird die Reaktion als kontrolliertes Ribonukleotid-Tailing (CTRT) bezeichnet.Vorzugsweise handelt es sich bei dem verwendeten Ribonukleotid nicht um CTP; besonders vorteilhaft ist die Verwendung von ATP.

Gegenstand der vorliegenden Erfindung sind auch Verfahren, bei denen das Produkt der Umsetzung anschließend mit einem weiteren doppelsträngigen Nukleinsäuremolekül, welches ein 3'-überhängendes Ende besitzt, das komplementär zum 3'-Ende des Produkts der Umsetzung ist, verbunden wird. Als weitere doppelsträngige Nukleinsäuremoleküle eignen sich beispielsweise DNA Vektoren oder aber kürzere Adaptor-Moleküle, welche Targetsequenzen für PCR Primer zur anschließenden Amplifikation aufweisen.

Außerdem können die eingesetzten doppelsträngigen Nukleinsäuremoleküle Sequenzen enthalten, die für eine weitere Analytik nach erfolgter Amplifikation hilfreich sind. Dazu gehören beispielsweise zur in-vitro Transkription geeignete Promotorsequenzen wie die prokaryotischen T7-, T3-, oder SP6-Promotoren oder auch Restriktionsschnittstellen, wobei selten auftretende Schnittstellen für sogenannte "rare cutter" Enzyme wie beispielsweise NotI besonders bevorzugt sind.

Vorzugsweise werden das weitere doppelsträngige Nukleinsäuremolekül sowie das 3'-Ende des Produkts der oben genannten Umsetzung miteinander ligiert. Dabei hat es sich herausgestellt, daß die Hybridisierung des Adaptors an die Erststrang-cDNA in Gegenwart von Standard-Ligationspuffern enthaltend DMSO besonders effizient ist, wobei Effizienzen von mehr als 90% Prozent erreicht werden können. Konzentrationen von 5-10 Vol.% DMSO haben sich als vorteilhaft herausgestellt. Bei der Optimierung der Reaktion wurde eine Konzentration von 7,5 Vol% ermittelt.

Dieser Schritt beinhaltet die eigentliche Selektion von full-length cDNA-Molekülen, welche aufgrund der Wahl von Adaptoren oder Vektoren stattfindet, die spezifische 3'-Überhänge besitzen. Diese Überhänge sind im wesentlichen dadurch gekennzeichnet, daß sie vollständig komplementär zum 3'-Ende der eingangs synthetisierten full-length cDNA sind. Dies wird dadurch erreicht, daß der Überhang im Falle einer Verwendung von ATP für die Terminale Transferase Reaktion von 5' nach 3' aus 3 oder 4 deoxy-Thymidin-Resten gefolgt von 3 deoxy-Guanosinresten besteht.

Aufgrund der Tatsache, daß während der Tailing-Reaktion der Reversen Transkriptase und während des kontrollierten Ribonukleotid-Tailings nicht an jedem cDNA-Molekül eine vollkommen identische Zahl an Nukleotidresten hinzugefügt wird, hat es sich als vorteilhaft erwiesen, wenn die eingesetzten Adaptoren aus einem Gemisch von Molekülen bestehen, deren 3 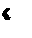 -Überhänge zwischen 3 und 4 deoxy-Thymidinresten sowie eventuell auch zwischen 3 bzw. 4 deoxy-Guanosinresten variieren.

Demzufolge ist auch ein Gemisch aus doppelsträngigen DNA-Adaptoren, enthaltend 3 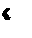 -Überhänge bestehend aus 5'-dT₃₋₄dG₃-3 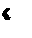 Gegenstand der Erfindung. Ebenfalls Gegenstand der Erfindung sind Erststrang-cDNAs, welche ein nicht mRNA-Template codiertes 3 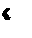 -Ende bestehend aus 5 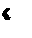 -dC₃₋₄-rA₃₋₄-3 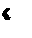 aufweisen.

Bei der sich erfindungsgemäß anschließenden Amplifikation mithilfe von PCR kann prinzipiell zwischen mehreren Möglichkeiten unterschieden werden:

### A) Konstruktion einer cDNA-Genbank

Zu diesem Zweck wird ein Primerpaar bestehend aus einem Adaptor-Primer und einem sogenannten Anker-Primer verwendet. Der verwendete Adaptor-Primer ist, wie oben beschrieben, gegen die doppelsträngige Sequenz des DNA-Adaptormoleküls gerichtet. Der Anker-Primer dagegen entspricht mit seiner Sequenz dem Ankerteil, welcher während der Erststrang-cDNA-Synthese durch die Reverse Transkriptase Reaktion an das 5'-Ende der cDNA angefügt wurde. Der auf diese Weise entstandene Pool von Amplifikationsprodukten enthält potentiell eine Vielzahl verschiedener full-length cDNAs und kann nach den gängigen molekularbiologischen Methoden zur Generierung einer cDNA-Genbank verwendet werden.

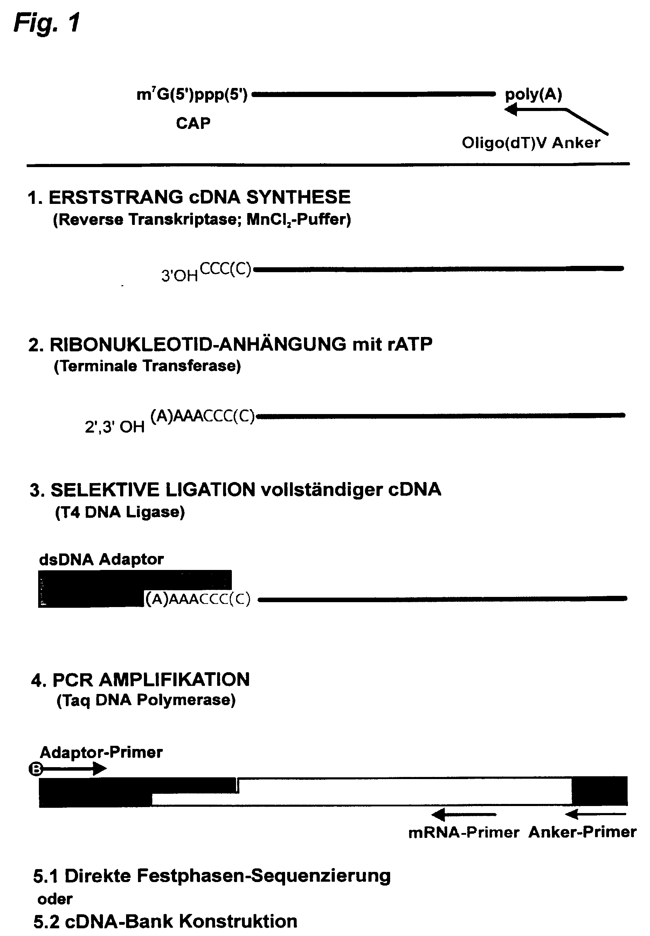

Ein schematischer Überblick über die erfindungsgemäße Methode findet sich in Abbildung 1: Die Methode vereint die 5 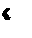 -CAP abhängige Addition von 3-4 Cytosinresten an den 3 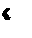 -Terminus von "full-length" Erststrang-cDNAs durch die Reverse Transkriptase (RT) (1., unter erfindungsgemäßer Verwendung eines Manganchlorid-haltigen Reaktionspuffers) mit der Technik "controlled ribonucleotide tailing of cDNA ends" (CRTC) durch das Enzym Terminale Transferase und rATP (2.). Aufgrund des so entstandenen terminalen Sequenzmotivs (5'-dC₃rA₃₋₄) kann die "full-length" cDNA selektiv an einen doppelsträngigen DNA-Adaptor (5'-dT₃₋₄ dG₃) unter Verwendung von *T4* DNA Ligase ligiert werden (3.). Diese Methode führt zur spezifischen Anreicherung von "füll-length" cDNAs die eine vollständige 5 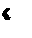 -Sequenz der mRNA enthalten. Nach einer geeigneten Amplifikation mithilfe von PCR kann im Anschluß daran eine cDNA-Bank konstruiert werden (5.2.).

### B) Isolierung und Amplifikation einer spezifischen mRNA, über die eine partielle Sequenzinformation vorliegt.

Als Primer zur Amplifikation werden in diesem Schritt ein sogenannter Adaptorprimer sowie ein mRNA-Primer verwendet. Der Adapterprimer ist gegen den doppelsträngigen Bereich des DNA-Adaptor-Moleküls gerichtet. Der mRNA-Primer dagegen ist komplementär zu einem vorbekannten Sequenzabschnitt der m-RNA. Die auf diese Weise erhaltenen Amplifikationsprodukte repräsentieren cDNAs, die an ihrem 5'-Ende vollständig sind, an deren 3'-Ende jedoch, je nach Wahl des mRNA-Primers, ein Sequenzabschnitt einer bestimmten Länge fehlt.

Für den Fall, daß es sich bei der zu identifizierenden mRNA um eine schwach exprimierte Spezies handelt, kann im Rahmen einer nested PCR mit einem weiteren Primerpaar, von denen wiederum ein Primer gegen die doppelsträngige Adaptor-Sequenz, sowie ein weiterer Primer gegen eine interne Sequenz der mRNA gerichtet ist, erneut amplifiziert werden.

In einer besonderen Ausführungsform kann der Adaptor-Primer eine immobilisierbare Markierung, wie beispielsweise Biotin, enthalten, so daß die entstehenden Amplifikationsprodukte an eine mit Streptavidin beschichtete Festphase gebunden werden können. Auf diese Weise können die Amplifikationsprodukte beispielsweise durch direkte Sequenzierung einfacher analysiert werden.

Gegenstand der Erfindung sind darüber hinaus auch Verfahren, bei denen die Amplifikation der cDNA mit einem mit einer immobilisierbaren Gruppe versehenen Primer durchgeführt wird. In einer alternativen Ausführungsform kann dagegen das doppelsträngige DNA-Adaptor-Molekül mit einer immobilisierbaren Gruppe versehen sein. Dadurch kann das abhängig von der cDNA generierte Amplifikationsprodukt zur weiteren Analytik an eine Festphase gekoppelt werden.

Gegenstand der Erfindung sind ferner insbesondere auch Verfahren, welche dadurch gekennzeichnet sind, daß die Amplifikationsreaktion mehrfach durchgeführt wird. Diese mehrfache Durchführung kann mit verschiedenen Amplifikationsprimern in Form einer nested PCR durchgeführt werden.

Gegenstand der Erfindung sind auch besondere Ausführungsformen des erfindungs-gemäßenVerfahrens, bei denen das Amplifikationsprodukt anschließend direkt sequenziert wird.

Über die genannten Möglichkeiten hinaus sind auch andere Amplifikationsmethoden Gegenstand der Erfindung, die auf speziellen Analyseformaten wie beispielsweise Differential Display (WO 93/18176), Serial Analysis of Gene Expression (US 5,695,937) subtraktiver Hybridisierung (Wang et al., Proc.Natl. Acad. Sci. USA 88: 11505 (1991)) oder linearer Amplifikation mithilfe von T7-RNA-Polymerase beruhen.

### Kurzbeschreibung der Abbildungen

- Abb. 1:: Übersichtsschema zur Methode der Cap-selektiven CRTC
- Abb. 2:: Erststrang cDNA-Synthese: 5 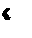 -CAP-abhängige Terminale Transferase Aktivität einer Reversen Transkriptase in Gegenwart von Manganchlorid
- Abb. 3:: 5 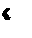 -CAP abhängige Terminale Transferase-Aktivität mit verschiedenen reversen Transkriptasen
- Abb. 4:: Mangan-Abhängigkeit der Terminalen Transferase-Aktivität von Reverser Transkriptase
- Abb. 5:: Kontrolliertes Ribonukleotid tailing mit rATP
- Abb. 6:: Selektive Ligation von full-length cDNA
- Abb. 7:: Erfindungsgemäße Amplifikation mit Hilfe von PCR
- Abb. 8:: Direkte Sequenzierung von erfindungsgemäß amplifizierter cDNA

### Beispiele

### Beispiel 1: 5'-CAP abhängige Terminale Transferase-Aktivität der SuperScript™ RT

Die Terminale Transferase-Aktivität der SuperScript™ RT (Gibco BRL) wurde unter den folgenden Bedingungen mit einer *in vitro* transkribierten 226nt RNA (β-Globin aus Kaninchen, GenBank V00882, Fragment (Pos. 633-1335) in pBlueScript KS kloniert zwischen KpnI und EcoRI, T3 Transkription, Termination durch NcoI-Verdau des Plasmids mit wahlweise mit nach Standardmethoden erzeugten 5 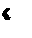 -CAP (1; 4), 5 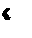 -Phosphat (2; 5) oder 5 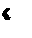 -OH (3; 6) Termini getestet.

| | |
|---|---|
| 10µl (8ng/µl) RNA | [89ng] |
| 1µl (1µM) Oligo (³²P-mark.) | [0.05µM each] |
| 4µl (5x) RT-Puffer | [50mM Tris-Cl 8.3, 75mM KCl, 3mM MgCl₂] |
| 1µl (100mM) DTT | [5mM] |
| 1µl (20x) MnCl₂-Puffer | [8mM MnCl₂,] |
| 1µl (10mM) dNTPs | [0.5mM] |
| 1µl (40u/µl) RNAse Inhibitor | [40units] |
| 1µl (200u/µl) SuperScriptII™ | [200units] |
| Gesamtvolumen: 20 µl | |

Als Oligonukleotid wurde ein β-Globin-spezifisches 5'-terminal 32P-markiertes 20mer verwendet (GenBank V000882, Nukleotid Pos. 661-680).

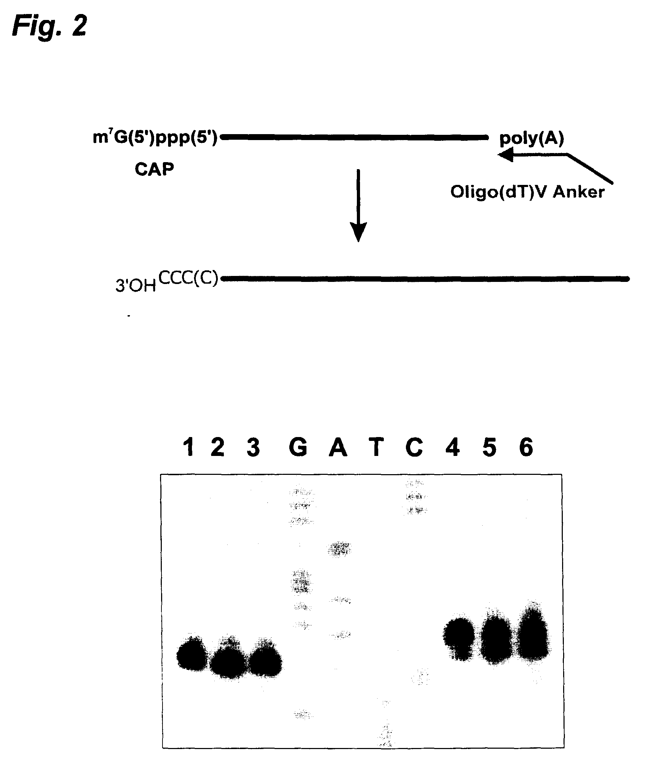

Vor Zugabe des Enzyms wurden die Reaktionsansätze 3 min. bei 80° C denaturiert und 3 min. bei 4° C abgeschreckt. Nach Zugabe des Enzyms wurde für 60min bei 42° C inkubiert. Anschließend wurden die Reaktionsprodukte auf einem 10% Polyacrylamid/8M-Harnstoff Gel aufgetrennt und auf einem PhosporImager quantifiziert. Als Längenkontrolle diente eine Sequenz, die mit demselben Oligonukleotid von einem klonierten Fragment des Globin-Gens generiert wurde. Das Ergebnis ist in Abb. 2 dargestellt. Im RT-Puffer ohne Mangan-Zusatz (1-3) ist die Transferase Aktivität auf die Addition eines Nukleotids beschränkt; die Reaktion erfolgt mit höherer Prozessivität bei einer mRNA Vorlage mit einem 5'-CAP Terminus (1, 73 % +1) im Vergleich zu 5 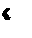 -OH (2, 57% +1) oder 5 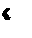 -Phosphat (3, 22% +1). Im Gegensatz dazu ist bei Verwendung eines Manganchlorid-haltigen Puffersystems (4-6) die Transferase Aktivität der reversen Transkriptase nicht nur abhängig von der CAP-Struktur am 5'-Terminus des mRNA Templates, sondern auch wesentlich effizienter. Der Anteil der Moleküle mit mehr als +2nt beträgt 91%. Bei 5 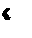 -CAP Termini (4) beträgt er 49% für +3nt und 42% für +4nt, gegenüber 57% +1nt und 39% +3nt bei 5 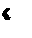 -Phosphat (5) und 40% +1nt, 22% +2nt und 34% +3nt bei 5 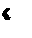 -OH Termini (6).

Alternativ dazu wurde Manganchlorid in einer Endkonzentration von 10 mM erst nach der cDNA Synthese hinzugesetzt, bevor die Reaktionsansätze für weitere 10 min inkubiert wurden. Dabei wurden in Bezug auf die Effizienz der Tailing-Reaktion keine signifikanten-Unterschiede im Vergleich zur Zugabe von Manganchlorid zu Beginn der Reaktion festgestellt.

### Beispiel 2: 5'-CAP abhängige Terminale Transferase-Aktivität: Vergleich verschiedener RTs

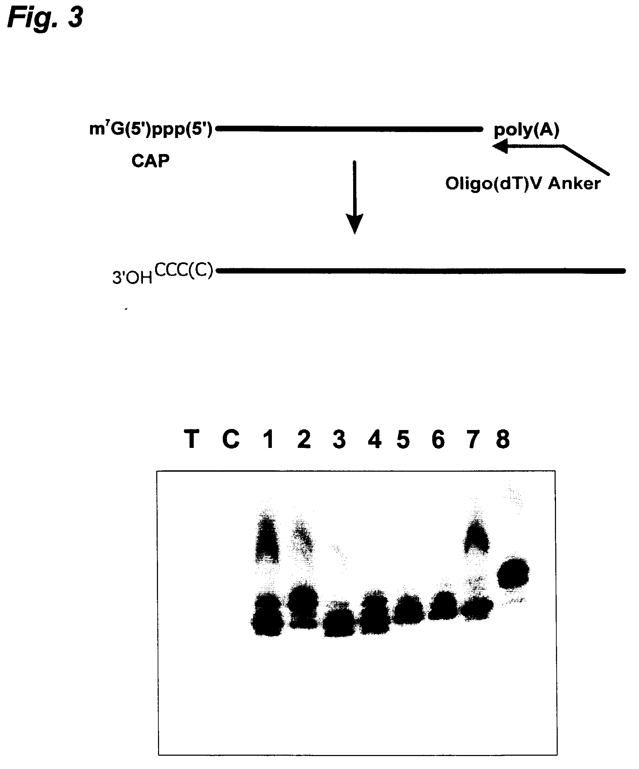

Das in Beispiel 1 dargestellte Experiment wurde mit verschiedenen Reverse Transkriptase Enzymen wiederholt. Das Ergebnis ist in Fig. 3 dargestellt. Dabei wurden RTs mit und ohne RNaseH-Aktivität [Mu-MLV (3,4) und AMV (5,6) bzw. Expand™ RT (1,2) und SuperScript™ (7, 8)] in bezug auf Terminale Transferase-Aktivität bei cDNA Synthese mit einer 5 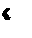 -CAP mRNA Vorlage miteinander verglichen. Dazu wurde der jeweilige Reaktionspuffer des Herstellers entweder original (1, 3, 5, 7) oder mit 8 mM Manganchlorid als Additiv (2, 4, 6, 8) getestet. Nur bei den RNaseH-defizienten Enzymen konnte eine Manganchlorid abhängige Transferase Aktivität festgestellt werden. Dabei unterscheiden sich SuperScript™ und Expand™ RT lediglich geringfügig bezüglich Effizienz im Einbau von 3 oder 4 Cytosinresten (Expand™ RT (2): +3 43%, +4 31%; SuperScript™ (8): +3: 8%, +4: 81%).

### Beispiel 3: Terminale Transferase-Aktivität der Expand™ RT: Mangan-Anhängigkeit

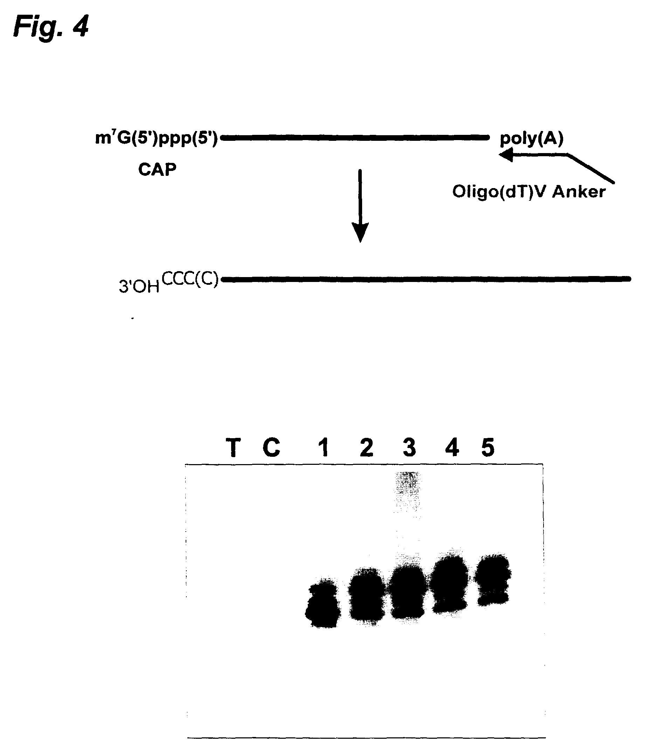

Das in Beispiel 1 beschriebene Experiment wurde wie in Fig. 4 dargestellt mit Expand Reverser Transkriptase™ (Roche Molecular Biochemicals) und verschiedenen Manganchlorid-Konzentrationen (0mM (1), 2mM (2), 4mM (3), 8mM (4), 10mM in einer zusätzlichen 15min Inkubation nach cDNA Synthese (5)) wiederholt. Dabei wurden folgende Effizienzen bezüglich des Einbaus eines 3. und 4. Nukleotids ermittelt: 23% (0mM), 43% (2mM), 63% (4mM), 73% (8mM), 70% (10mM, Post-Inkubation).

### Beispiel 4: rATP-tailing mit terminaler Transferase ("Ribo-tailing")

Nach einer geeigneten Aufreinigung (Roche Molecular Biochemicals High pure Purification Kit) wurde die erfindungsgemäß synthetisierte cDNA aus Beispiel 1 in folgendem Reaktionsansatz für 30 min bei 30min 37°C inkubiert:

| | |
|---|---|
| 20µl cDNA | |
| 6µl (5x) TdT-Puffer | [200mM K-Cacodylate, 25mM Tris-Cl 6.6, 0.25mg/ml BSA] |
| 3µl (25mM) CoCl₂ | [2.5mM] |
| 0.5µl (10mM) ATP | [167µM] |
| 0.5µl (25u/µl) Terminale Transferase | [12.5units] |
| Gesamtvolumen: 30 µl | |

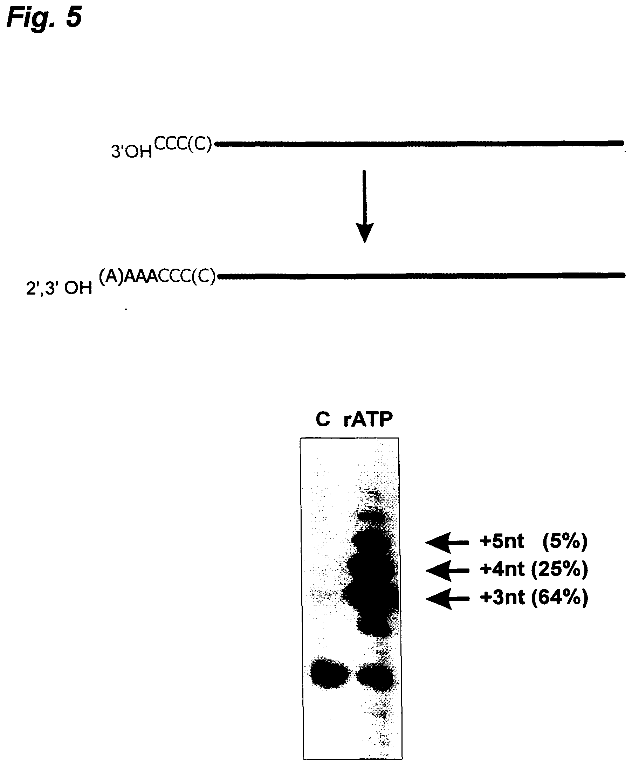

Zur Analyse der Effizienz der Reaktion wurde das Experiment anstelle von cDNA mit einem ³²P-markierten deoxy-Oligonukleotid (20mer) bei gleichen Reaktionsbedingungen wiederholt. Das Ergebnis ist in Fig. 5 dargestellt: Die Reaktionsprodukte wurden auf einem 20% Polyacrylamid/8M-Harnstoff Gel aufgetrennt und auf einem PhosporImager quantifiziert. Dabei wurde die Gesamteffizienz von 94% der Reaktion in Bezug auf die Addition von 3 (64%), 4 (25%) oder 5 (5%) Adenosin-Resten ermittelt.

### Beispiel 5: Selektive Ligation an den Adaptor

Nach einer Standard-Ethanolpräzipitation wurde die zuvor mit Terminaler Transferase behandelte cDNA in 15µl Wasser resuspendiert und mit einer Population von Adaptormolekülen ligiert: Die Adaptoren bestanden aus einem doppelsträngigen Bereich von 50 Basenpaaren mit einem 6-7-Nukleotid*'*-3 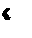 Überhang von [5'-T₃₋₄ G₃-3 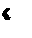 ]. Der Ligationsansatz setzte sich wie folgt zusammen:

| | | |
|---|---|---|
| 13µl | ATP-tailed cDNA | |
| 2µl (50ng/µl) | 56/57bp-Adaptor | [100ng, 3pmol] |
| 2µl (10x) | Ligationspuffer | [66mM Tris-Cl 7.5, 5mM MgCl₂, 1mM DTT, 1mM ATP] |
| 1.5µl | DMSO | [7.5 Vol %] |
| 1.5µl (1u/µl) | *T4* DNA Ligase | [1.5units] |
| Gesamtvolumen: 20 µl | | |

Nach Inkubation für 5 min 37°C und 2min bei 24°C zur selektiven Hybridisierung des Adaptorüberhangs an das 3 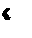 - getailte Ende der cDNA wurde der Ansatz für 16h bei 16°C zur Ligation inkubiert.

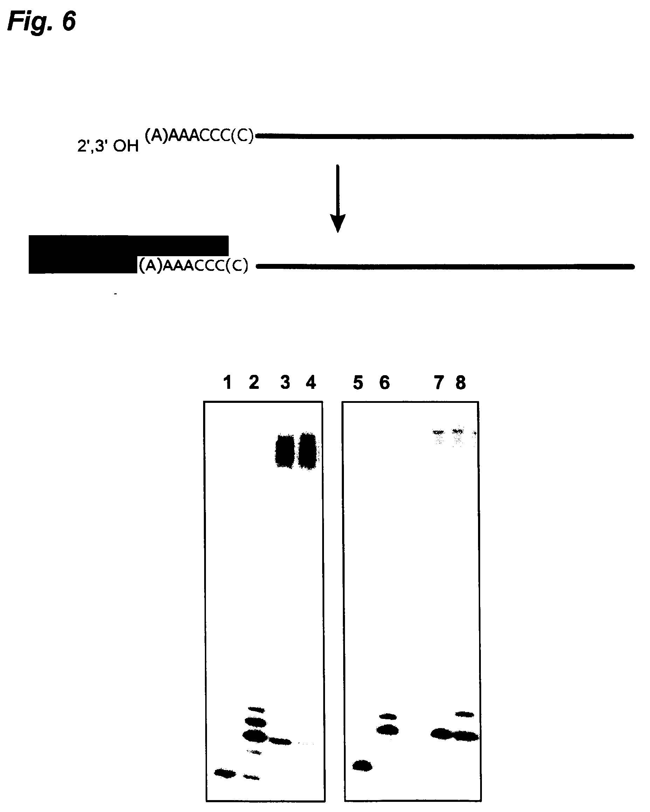

Die Selektivität der Ligation an den Adaptor wurde ebenfalls in einem Assay mit ³²P- markiertem Oligonukleotid (20-mer) getestet. Das Ergebnis ist in Fig. 6 dargestellt. Dabei wurden Oligonukleotide mit "kompatiblen" (3'- CCC, 1-4) und "inkompatiblen" (3'- AGC, 5-8) 3'-Termini verglichen. Die Oligonukleotide (1 bzw. 5) wurden nach rATP-Tailing wie in Beispiel 4 beschrieben (1 bzw. 6) in einem Standard-Ligationsansatz mit *T4* DNA Ligase (3 bzw. 7) oder mit DMSO (7.5% v/v) als Additiv (4 bzw. 8) an den Adaptor [5'-G₃T_{3*-*4}] ligiert. Die Reaktionsprodukte wurden in einem 15% Polyacrylamid/8M-Harnstoff Gel aufgetrennt und auf einem PhosporImager quantifiziert. Durch die Addition von DMSO (4) werden 93% (gegenüber 77% unter Standardbedingungen, 3) der cDNA Fraktion mit kompatiblen 3'-Termini ligiert. Darüber hinaus sind die Ligationsbedingungen selektiv, d.h. selbst Moleküle, die mit einem C terminieren, werden unter diesen Bedingungen nur zu 14% (7) bzw. nur zu 8% (8) bei Verwendung von DMSO im Puffer ligiert. Dadurch wird die selektive Anreicherung von vollständigen cDNAs erhöht.

### Beispiel 6: Amplifikation einer erfindungsgemäß hergestellten und modifizierten, humanen GAPDH-cDNA im Vergleich zur Amplifikation durch 5'-RACE

Ausgehend von unterschiedlichen Mengen einer poly-A+ mRNA (10ng, 2,5 ng, 0,5 ng, 100 pg, 20 pg) isoliert aus der humanen Zelllinie MOLT-3 (ATCC-Stammsammlung No. CRL-1552) wurde gemäß Beispielen 1, 4 und 5 eine erfindungsgemäße cDNA-Synthese mit deoxy-Cytosin-Tailing, eine terminale Transferase Reaktion mit rATP sowie eine Ligation mit einem erfindungsgemäßen Adaptor durchgeführt.

Anschließend wurde zur spezifischen Amplifikation von GAPDH eine PCR-Reaktion unter folgenden Bedingungen durchgeführt:

| | | |
|---|---|---|
| 2µl | Aliquot der Ligation aus Beispiel 5 | |
| 1µl (50µM) | Primer STI-2 | [1µM] |
| 1µl (50µM) | Primer GAPDH-2/M | [1µM] |
| 1µl (10mM) | dNTPs | [0.5mM] |
| 5µl (10x) | Expand™-Puffer | [Roche Molecular Biochemicals, enthaltend 1.5mM Mg⁺⁺] |
| 39.5µl | H₂O | |
| 0.5µl (3.5u/µl) | Expand™ | [Roche Molecular Biochemicals] |
| Gesamtvolumen: 50 µl | | |

| | | | |
|---|---|---|---|
| Thermozyklen:: | 1x | 30sec | 72°C |
| | 1x | 3min | 94°C |
| | 35x | 20sec | 94°C |
| | | 30sec | 64°C |
| | | 40sec | 72°C |
| | 1x | 5min | 72°C |

Dabei handelte es sich bei GAPDH-2M um einen 24mer entsprechend Nukleotidposition 83-106 aus Exon 4 (GenBank Acc. No. J04038) des GAPDH-Gens mit einem errechneten Schmelzpunkt Tm von ca. 70°C. Der STI-2 Primer besaß eine Länge von 20 deoxy-Nukleotiden und einen errechneten Schmelzpunkt Tm von ca 69°C. In parallelen Ansätzen wurde unter Verwendung der gleichen Amplifikationsprimer GAPDH gemäß dem Amplifikationsprotokoll des aus dem Stand der Technik bekannten 5 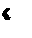 -RACE Verfahrens (Frohmann, M. (1994) PCR Methods and Applications, 4, 540-558) amplifiziert.

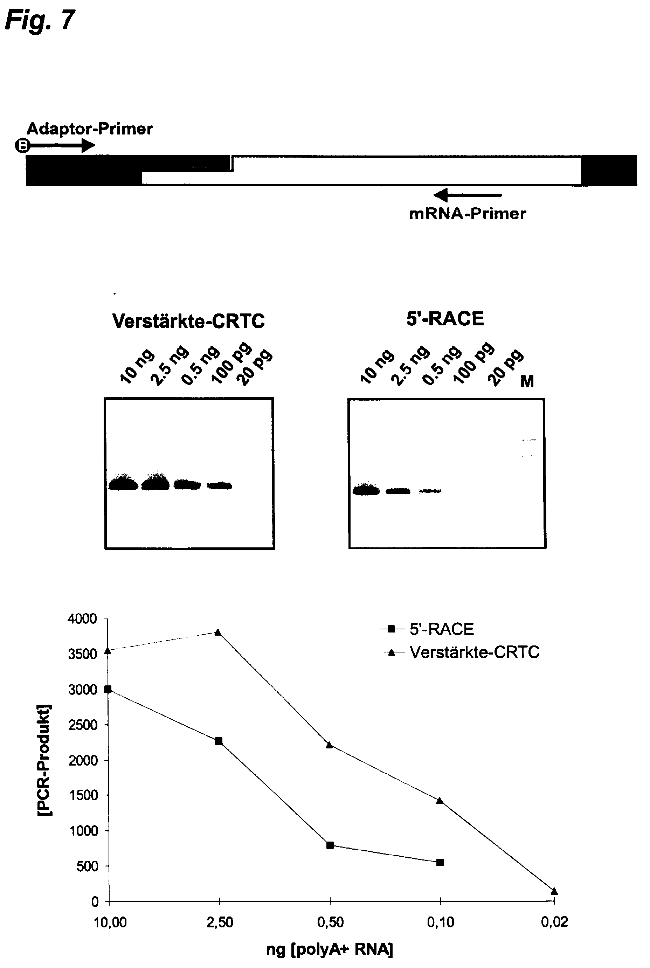

Die PCR-Produkte wurden in einem 1.25% Agarosegel aufgetrennt und densitometrisch ausgewertet. Wie dem in Fig. 7 dargestellten Ergebnis zu entnehmen ist, ist die die Sensitivität der erfindungsgemäßen Methode im Vergleich zu 5 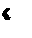 -RACE gemäß dem Stand der Technik deutlich erhöht.

### Beispiel 7 Direkte Sequenzierung des PCR-Amplifikats nach Enhanced-CRTC

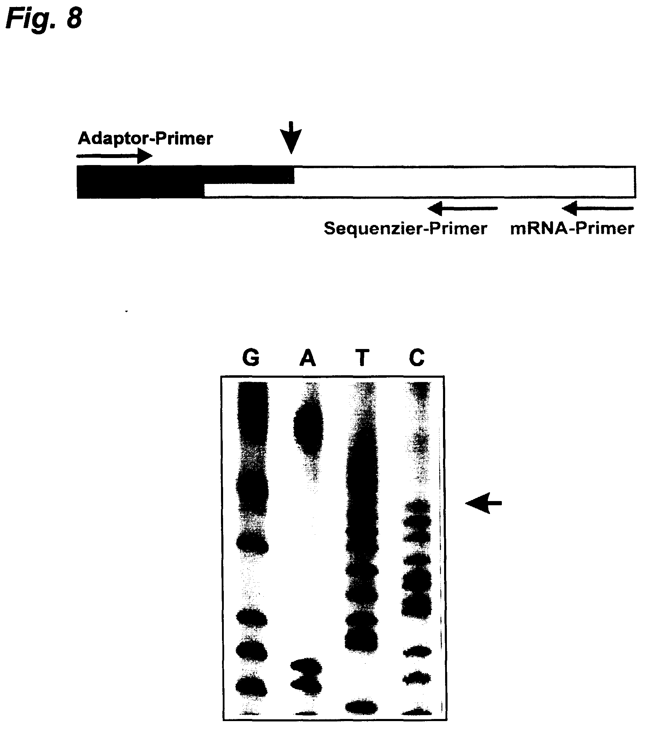

Das erfindungsgemäß erhaltene Amplifikationsprodukt aus Beispiel 6 wurde entsprechend dem Prinzip der dideoxy-Methode wie in Schmitt und Müller, Nucl. Acids Res. 24, 1789-1791 (1996) beschrieben, mit einem IRD800-Fluoreszenz-markiertem GAPDH Primer entsprechend Nukleotidposition 78 bis 97 aus Exon 3 des GAPDH Gens (GenBank Acc. No. J04038, Ercolani L., Florence B., Denaro M., Alexander M.; "Isolation and complete sequence of a functional human glyceraldehyde-3-phosphate dehydrogenase gene"; J. Biol. Chem. 263:15335-15341(1988)). in Richtung des Transkriptionsstarts sequenziert. Wie in Fig. 8 dargestellt, erlaubt die Sequenz die exakte Zuordnung des Transkriptionsstarts (Pfeil) des humanen GAPDH-Gens.

## Patentansprüche

1. Verfahren zur Modifikation, Klonierung oder Amplifikation einer cDNA, dadurch gekennzeichnet, daß die Erststrang-cDNA-Synthese in Gegenwart von Magnesium²⁺-Ionen und Mangan²⁺-Ionen durchgeführt wird.

2. Verfahren zurModifikation, Klonierung oder Amplifikation einer cDNA, dadurch gekennzeichnet, daß direkt nach der Erststrang-cDNA-Synthese die cDNA in Gegenwart von Mangan²⁺-Ionen inkubiert wird.

3. Verfahren gemäß Anspruch 1-2, dadurch gekennzeichnet, daß die Mangan²⁺-Ionen-Konzentration während der cDNA-Synthese mindestens 1 mM aber höchstens 20 mM ist.

4. Verfahren gemäß Anspruch 1 bis 3, dadurch gekennzeichnet, daß im Anschluß an die cDNA-Synthese die cDNA in Anwesenheit von terminaler Transferase mit einem Ribonukleotid-Triphosphat oder einem modifizierten Nukleotid-Triphosphat umgesetzt wird.

5. Verfahren nach Anspruch 4, dadurch gekennzeichnet, daß das Produkt der Umsetzung mit einem weiteren doppelsträngigen Nukleinsäuremolekül mit einem 3' überhängenden Ende, welches komplementär zum 3' Ende des Produkts der Umsetzung ist, verbunden wird.

6. Verfahren nach Anspruch 5, dadurch gekennzeichnet, daß das weitere Nukleinsäuremolekül ein DNA Vektor- oder ein Adaptormolekül ist.

7. Verfahren nach Anspruch 5 bis 6, dadurch gekennzeichnet, daß das weitere Nukleinsäuremolekül an das 3'-Ende des Produkts der Umsetzung ligiert wird.

8. Verfahren nach Anspruch 7, dadurch gekennzeichnet, daß die Ligation in Gegenwart von 5 bis 10 Vol% DMSO durchgeführt wird

9. Verfahren nach Anspruch 8, dadurch gekennzeichnet, daß die spezifische Ligation mit einer Effizienz von größer als 90 % durchgeführt wird.

10. Verfahren nach einem der Ansprüche 7 bis 9, dadurch gekennzeichnet, daß die Amplifikation mit einem mit einer immobilisierbaren Gruppe versehenen Primer amplifiziert wird.

11. Verfahren nach Anspruch 7 bis 9, dadurch gekennzeichnet, daß das verwendete doppelsträngige DNA-Adaptermolekül mit einer immobilisierbaren Gruppe v2ersehen ist.

12. Verfahren nach Anspruch 10 bis 11, dadurch gekennzeichnet, daß die Amplifikationsreaktion mehrfach durchgeführt wird.

13. Verfahren nach Anspruch 7 bis 12, dadurch gekennzeichnet, daß eine nested PCR durchgeführt wird.

14. Verfahren nach Anspruch 10 bis 12, dadurch gekennzeichnet, daß das Amplifikationsprodukt direkt sequenziert wird.

15. Gemisch aus doppelsträngigen DNA-Adaptoren, enthaltend 3 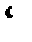 -Überhänge bestehend aus 5'-dT₃₋₄dG₃-3 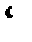 .

16. Erststrang-cDNA gekennzeichnet durch ein nicht mRNA-Template codiertes 3 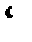 -Ende bestehend aus 5 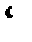 -dC₃₋₄-rA₃₋₄-3 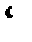 .
